Europäisches Patentamt

(19) European Patent Office

Office européen des brevets

(11) Publication number: **0 213 748**
**B1**

## (12) EUROPEAN PATENT SPECIFICATION

(45) Date of publication of the patent specification:
12.12.90

(51) Int. Cl.⁵: **A61M 29/02**

(21) Application number: 86305873.1

(22) Date of filing: 30.07.86

(54) Dual dilatation catheter assembly and miniature balloon dilatation catheter for use therewith.

(30) Priority: 30.07.85 US 760636

(43) Date of publication of application:
11.03.87 Bulletin 87/11

(45) Publication of the grant of the patent:
12.12.90 Bulletin 90/50

(84) Designated Contracting States:
AT CH DE FR GB IT LI NL

(56) References cited:
EP-A- 0 054 357
GB-A- 2 127 294
US-A- 4 295 464
US-A- 4 299 226

(73) Proprietor: ADVANCED CARDIOVASCULAR SYSTEMS,
INC., 1395 Charleston Road, Mountain View,
CA 94039-7101(US)

(72) Inventor: Samson, Wilfred Joseph, 19691 Falwell
Avenue, Saratoga California 94070(US)
Inventor: Williams, Ronald G., 49 Showers Drive N 164,
Mountain View California 94040(US)
Inventor: Mar, Craig Evan, 704 San Carlos Court,
Fremont California 94539(US)

(74) Representative: Bizley, Richard Edward et al, BOULT,
WADE & TENNANT 27 Furnival Street, London
EC4A 1PQ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a dual dilatation catheter assembly and to a miniature balloon dilatation catheter for use therewith.

Balloon dilatation catheters have heretofore been provided. However, it has been difficult with such catheters to dilate stenoses in blood vessels which have very small openings therein. There is therefore a need for a dilatation catheter which can be utilized for such situations.

US-A 4, 299, 226 discloses apparatus for dilating coronary arteries which comprises a single lumen guide catheter and a double lumen balloon dilating catheter to be inserted into the guide catheter. The balloon dilating catheter comprises an inner and outer tubular elements forming an inner lumen and an outer annular lumen.

A guide wire extends through the inner lumen and a balloon is carried by the outer tubular element.

The guide catheter is inserted in the blood vessel until the end is adjacent to an obstruction and then the dilating catheter is inserted until the balloon projects from the distal end of the guide catheter and is in the obstruction. The balloon is then inflated to enlarge the blood vessel.

EP-A 0, 054, 357 discloses an inflating and deflating device for use with a vascular dilating balloon catheter, which comprises a cylinder having a T-shaped handle, a piston in the cylinder connected by a rod to a handle, a pressure regulator, a stop cock between the cylinder and the regulator, a pressure gauge connected to an outlet of the pressure regulator and a tube connected to another outlet of the pressure regulator for connection to a catheter.

The T-shaped handle enables the balloon to be inflated using one hand. To obtain the desired amount of radiographic contrast liquid, the piston is moved to the indicated volume, and then the piston is used to pressurise a container containing the liquid to force it into the device.

In one aspect, the invention provides a dilatation catheter assembly for dilating a stenosis in a blood vessel, which assembly comprises a first dilatation catheter having a first tubular element with a first lumen extending therethrough, a second tubular element extending around the first tubular element and forming an annular second lumen with the first element and first a balloon which is carried by the second tubular element and is in communication with the second lumen, the dilatation catheter assembly being characterised by a second dilatation catheter extending through the first lumen, the second dilatation catheter having a flexible elongate tubular element with a third lumen extending therethrough, a second balloon which is carried by the flexible tubular element and is in communication with the the third lumen and a guiding member which extends through the second balloon, the second balloon having a collapsed diameter which is less than the inner diameter of the first lumen of the first dilatation catheter whereby the second dilatation catheter can be inserted into and removed from the first dilatation catheter.

In another aspect the invention provides a miniature balloon dilatation catheter for dilating a stenosis in a blood vessel, with a deflated diameter not greater than 0.59mm (0.023 inch), the catheter having a flexible elongate tubular element with a lumen extending therethrough, a balloon which is carried by the flexible elongate tubular element and is in communication with the lumen, a guiding member extending through both the lumen and the balloon, a flexible body secured to the distal end of the balloon and disposed about the portion of the guiding member extending out of the distal end of the balloon the guiding member being secured to the tip of said flexible body, and means for inflating the balloon with a predetermined quantity of fluid.

In the accompanying drawings:

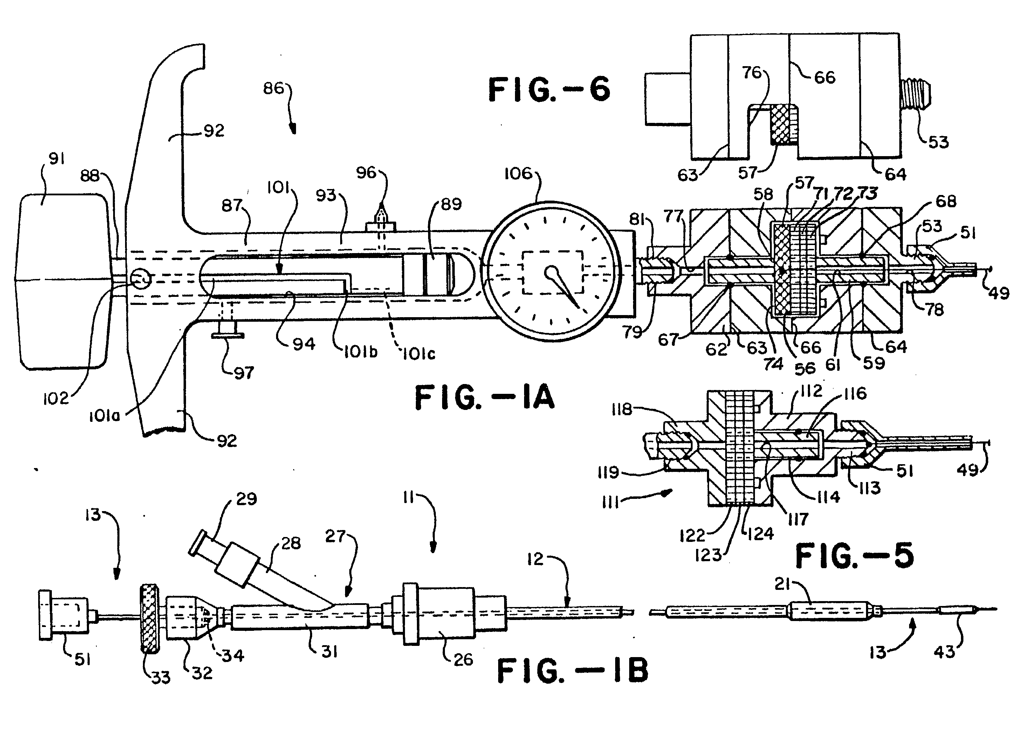

Figures 1A and Figure 1B show a side elevational view of a dual dilatation catheter assembly;

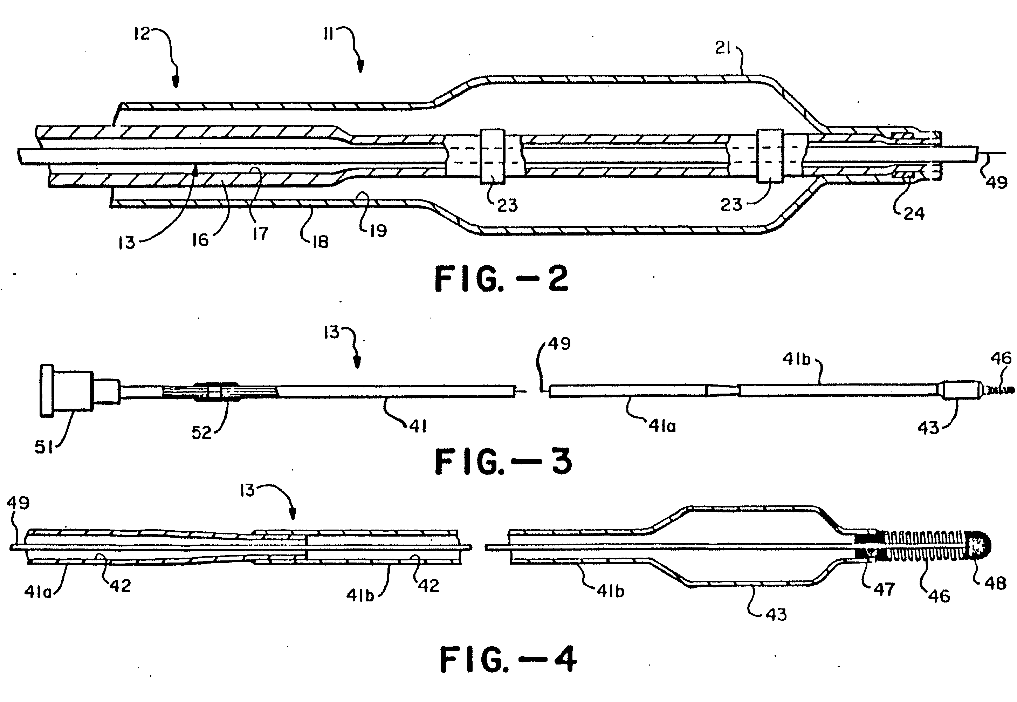

Figure 2 is an enlarged longitudinal sectional view of a portion of the catheter assembly shown in Figures 1A and 1B;

Figure 3 is a view partly in longitudinal section of the miniature balloon dilatation catheter assembly shown in Figures 1A and 1B;

Figure 4 is an enlarged longitudinal sectional view of the distal extremity of the miniature balloon dilatation catheter shown in Figure 3;

Figure 5 is a side elevational view of the rotation limiting device shown in Figure 1A: and

Figure 6 is a partial cross-sectional view showing another rotation limiting device.

As shown on the drawings, a dual catheter assembly 11 comprises a first dilatation catheter 12 and a second or miniature dilatation catheter 13.

The first dilatation catheter 12 comprises a first flexible elongate tubular element 16 having a lumen 17 extending therethrough and a second flexible elongate tubular element 18 which extends around the first tubular element and is coaxial therewith. An annular lumen 19 is formed between the first and second tubular elements 16 and 18 and extends longitudinally thereof. A balloon 21 is carried by the second tubular element and is formed integral therewith and has its interior in communication with the lumen 19 extending between the first and second tubular elements 16 and 18.

Means is provided for visually indicating the distal and proximal extremities of the balloon 21 and comprises a pair of bands 23 formed of a suitable material, for example, gold which are carried on the exterior surface of the first tubular element 16. An additional band 24 is carried by the distal extremity of the first and second tubular elements 16 and 18 and is disposed between them. The first and second tubular elements 16 and 18 are formed of a suitable plastic material, for example, polyethylene. The outer or second tubular element 18 is formed of a heat shrinkable polyethylene and its distal extremity is shrunk onto the distal extremity of the first tubular element 16 to form a liquid-tight seal.

The proximal extremities of the first and second tubular elements 16 and 18 are mounted in a rotary joint 26. A side arm adapter 27 is mounted on the rotary joint 26 and is provided with a side arm 28 which is provided with a Luer fitting 29. The side arm 28 is in communication with the lumen 19 formed between the first and second tubular elements. The first tubular element 16 extends through the central or main arm 31 of the side arm adapter 27 and extends into a fitting 32 which is provided with a thumb screw 33. The thumb screw 33 is adapted to engage an O-ring 34 to form a liquid-tight seal with respect to the second dilatation catheter 13 which extends therethrough and which extends through the lumen 17 of the first tubular element 16 of the first dilatation catheter 12.

The first dilatation catheter 12 has suitable dimensions. For example, the catheter 12 itself can have a length of approximately 135cm. The first tubular element 16 can have an outer diameter of 1.02mm (0.040 inches) and an inner diameter of 0.76mm (0.030 inches). The necked down portion of the element 16 within the balloon 21 can have an outside diameter of 0.74mm (0.029 inches) and an inside diameter of 0.59mm (0.023 inches). The second tubular element 18 can have an outside diameter of 1.42mm (0.056 inches) and an inside diameter of 1.17mm (0.046 inches). The balloon 21 can have a length of 25mm and the distal extremity of the first and second tubular elements 16 and 18 can have a length of approximately 9mm. The distal extremity of tip of the first dilatation catheter 12 can have a maximum diameter of 1.14mm (0.045 inches). The balloon 21 can have a diameter of 3mm.

The second or miniature dilatation catheter 13 comprises a flexible elongate tubular element 41 which is formed of several sections 41a and 41b and through which a lumen 42 extends. The section 41a is formed of stainless steel having an outside diameter of approximately 0.35mm (0.014 inches) with a wall thickness of 0.08mm (0.003 inches) so that the inside diameter is approximately 0.20mm (0.008 inches). The distal extremity of the portion 41a is ground down to a smaller dimension as, for example, an outside diameter of 0.25mm (0.010 inches). The exterior surface of the portion 41a is coated with a low friction material such as PTFE.

The other portion 41b of the flexible elongate tubular element 41 comprises a plastic tube of a suitable type for example polyethylene of a length ranging from 20 to 30cm. This tube has an outside diameter of 0.27mm (0.014 inches) and is mounted by friction fit on the distal extremity of the portion 41a. A balloon 43 is carried by the portion 41b and is formed integral therewith and has its interior in communication with the lumen 42. The balloon 43 can have a suitable outside diameter such as 1.8mm and a suitable length such as approximately 1.5cm.

A coil 46 formed of a suitable material, for example a platinum/tungsten alloy has its proximal extremity secured to the distal extremity of the portion 41b of the tubular element 41 by suitable means such as an adhesive 47. A semi-spherical tip 48 formed of a suitable material, for example gold is provided on the distal extremity of the coil 46. A guide wire 49 of a suitable diameter such as 0.13mm (0.005 inches) extends through the lumen 42 and through the balloon 43 and is bonded to the distal extremity of the

portion 41b by adhesive 47 and also to the tip 48. The guide wire 49 extends to the proximal extremity of the tubular element 41 and extends through a fitting 51 which is secured to the tubular element 41 by suitable means for example solder 52. The second or miniature dilatation catheter can have a suitable length, for example 175cm.

The fitting 51 is adapted to be threaded in a threaded extension 53 of a rotation limiting device 56. The guide wire 49 extends through and is secured to a knob 57 of the rotation limiting device 56 by a set screw 58.

The knob 57 is rotatably mounted on a rigid tube 59 which has a flow passage 61 extending therethrough. The rigid tube 59 is mounted in a cylindrical block 62 which is provided with parting lines 63, 64 and 66. The parting lines 63 and 64 are provided in the block 62 at the points where O-rings 67 and 68 are disposed. The O-rings 67 and 68 provide liquid-tight seals between the block 62 and the rigid tube 59. The parting 66 is provided to facilitate the insertion of the torque knob 57 as well as three rotation limiting disks 71, 72 and 73.

Each of the disks, 71, 72 and 73, as well as the torque knob 57, is provided with an extension (not shown) which extends into an arcuate recess provided in the adjacent disk. The disks 71, 72 and 73 are rotatably mounted upon the rigid tube 59 and permit limited rotation, as for example, three complete rotations of the torque knob 57 with respect to the block 62.

The block 62 is provided with a cylindrical recess 74 which receives the knurled knob 57 as well as the disks 71, 72 and 73. A cut-out 76 is also provided in the block 62 and facilitates engagement of the knurled knob 57 by one of the fingers of the hand. The block 62 is provided with flow passages 77 and 78 which are in communication with the flow passage 61 provided in the rigid tube 59. The block 62 is provided with an internally threaded bore 79 which receives an externally threaded extension 81 which forms a part of a hand-operated inflating device 86 of a type which can utilize a gas, for example carbon dioxide, for inflating the balloon 43. The inflating device 86 comprises a housing 87 which has a piston rod 88 slidably disposed therein which carries a piston 89. The piston rod 88 is adapted to be operated by a button 91 carried by the proximal extremity of the rod. The housing 87 carries a pair of side arms 92 which are adapted to be engaged by the fingers of the hand while the button 91 is engaged by the palm of the same hand so that the piston rod 88 can be pushed inwardly. The piston 89 is slidably mounted in a cylinder 93 which is carried by the housing 87 and a portion of which can be viewed through the elongate openings 94 provided on opposite sides of the housing 87. A one-way valve 96 of a suitable type such as a duckbill valve is mounted on the cylinder 93 in a suitable position, for example, at a location in which three cubic centimeters of volume are available from the one-way valve 96 to the distal extremity of the cylinder 93. An inlet fitting 97 is mounted on the cylinder 93 adjacent to the proximal extremity of the cylinder in a position which is rotated by a suitable angle for example 90° to 180° from the position where the one-way valve 96 is located.

Co-operative means is carried by the piston rod 88 and the cylinder 93 so as to prevent uncovering of the inlet fitting 97 except at a predetermined angular position of the piston rod 88 with respect to the cylinder 93. For this purpose, an elongate slot 101 is provided in the piston rod 88 which extends from the button 91 to near the piston 89. The slot 101 comprises a portion 101a which extends from the button 91 for a substantial length of the piston rod 88 and adjoins a portion 101b which extends at right angles to the portion 101a. The portion 101b adjoins a further portion 101c which extends at right angles to the portion 101b and is parallel to and extends away from the portion 101a. A pin 102 is provided in the housing 87 and is seated in the slot 101 and limits the movement of the piston rod 88 as hereinafter described. A pressure gauge 106 is mounted on the distal extremity of the housing 87 and is in communication with the cylinder 93 and with the externally threaded extension 81.

Operation of the miniature or second dilatation catheter 13 in conjunction with the first dilatation catheter 12 in the dual dilatation catheter assembly 11 is now briefly described as follows. Let it be assumed that the first dilatation catheter 12 has been inserted into a vessel of a human being and that the stenosis which has been located is too small for the balloon 21 to enter.

The second balloon dilatation catheter 13 is prepared for use. A suitable source of carbon dioxide, for example, a 50°C syringe (not shown) of carbon dioxide is placed in the fitting 97 of the inflating device 86. While holding the side arms 92 in one hand, the button 91 is grasped with the other hand and the piston rod 88 is retracted through the length of the portion 101a of the slot 101. When the end of the portion 101a is reached, the button 91 is rotated through 90° so that the pin 102 travels in the portion 101b. As soon as this travel has been accomplished, the piston rod 88 can be further retracted by the pin 102 travelling in the portion 101c of the slot. This movement of the pin 102 in the portion 101c permits the piston 89 to clear the inlet fitting 97.

The cylinder 93 is then filled and purged with the 50 cubic centimeters of carbon dioxide with the excess carbon dioxide escaping through the one-way valve 96. As soon as purging with the carbon dioxide has been accomplished, the inflating device 86 is grasped by the hand and the piston rod 88 is pushed inwardly by the fingers of the hand grasping the side arms 92 and the palm of the same hand engaging the button 91. Travel of the piston 89 occludes the inlet 97 so that further carbon dioxide gas cannot be introduced into the cylinder 93. The button 91 is then rotated through 90° to cause the pin 102 to travel in the portion 101b of the slot. Thereafter, the piston rod 88 is advanced so that the pin 102 travels in the portion 101a of the slot 101. As the piston moves forwardly, excess carbon dioxide is discharged through the one-way valve 96. This discharge continues until the piston 89 reaches the one-way valve 96 and occludes it.

As soon as the purging operation has been completed as hereinbefore described, the second dilatation catheter 13 is ready to be introduced into the first dilatation catheter 12. For this purpose, the first dilatation catheter 12 is removed a short distance so that it is away from the stenosis which is to be dilated and the guide wire, if one has been used for positioning the first dilatation catheter 12, is removed. The second dilatation catheter 13 can now be inserted through the fitting 32. The advance of the second dilatation catheter 13 can be facilitated by use of the torque knob 57 to cause rotation of the guide wire 49 which in turn can be utilized for causing movement of the spring-like tip provided by the coil 46. So that undue stress is not placed on the coil 46 or undue wrapping of the balloon 43 does not occur, the rotation limiting device 56 limits the rotation to not exceed, for example, three or four turns. After the tip 48 of the second dilatation catheter 13 has been advanced into the stenosis, and the balloon 43 positioned properly, the balloon 43 can be inflated. The piston rod 88 is then depressed by grasping the side arms 92 by the fingers and the button 91 by the palm of the same hand to push it inwardly. As the piston 89 is advanced, the carbon dioxide within the cylinder 93 will be discharged through the one-way valve 96.

This continues until the piston 89 occludes the one-way valve 96 so that there remains in the cylinder a predetermined volume of carbon dioxide gas, for example, approximately three cubic centimeters. Further depression of the piston rod 88 and the piston 89 thereby causes this remaining three cubic centimeters of carbon dioxide gas to be introduced into the balloon 43 to inflate the balloon to dilate the stenosis. By utilizing carbon dioxide very substantial pressures up to 1.38 M Pascals (200 psi) can be generated. Gas, rather than a liquid, is utilized as an inflating medium for the balloon 43 because of the very small diameter of the passageway or lumen 42 extending into the balloon 43. Carbon dioxide is preferable because it can be more readily assimilated in the blood stream.

After the stenosis has been widened sufficiently, the second dilatation catheter 13 can be removed from the stenosis and also from the first dilatation catheter 12. Thereafter, if necessary the guide wire can be reintroduced into the first dilatation catheter 12 and the first dilatation catheter advanced into the stenosis until the balloon 21 comes into alignment with the stenosis.

Thereafter the balloon 21 can be operated by utilizing a radiographic contrast liquid which is introduced through the side arm 28. After the balloon 21 has been inflated one or more times to dilate the stenosis, the first dilatation catheter 12 can be removed.

By limiting the quantity of carbon dioxide which can be introduced into the balloon 43, the amount of gas present in the balloon 43 is insufficient to cause damage to the patient in the event of rupture of the balloon. Thus it can be seen that the dual catheter assembly can be utilized safely and can be utilized for penetrating stenoses having minimal openings. If desired, the miniature dilatation catheter 13 can be utilized by itself. Since a guide wire 49 has been provided in conjunction with the second dilatation catheter 13, the tip or coil 46 can be provided with a slight bend and can be rotated through a number of rotations as determined by the rotation limiting device 56 to facilitate guiding the second dilatation catheter 13 to the desired location. This rotation capability of the second dilatation catheter 13 can also be utilized when it is being used in conjunction with the first dilatation catheter 12 as hereinbefore explained.

Figure 5 shows another rotation limiting device 111 which comprises a movable body 112 which is provided with an externally threaded extension 113 which is threaded into the fitting 51. The movable body 112 is provided with a cylindrical recess 114 which receives a stem 116 having a flow passage 117 extending therethrough. The stem 116 is formed integral with a fixed body 118 through which the flow passage 117 extends. The fixed body 118 is provided with a threaded bore 119 which receives the externally threaded extension 81 of the inflating device 86. Three rotatable disks 122, 123 and 124 are provided which are rotatably mounted upon the stem 116. These disks 132, 123 and 124 are provided with protrusions (not shown) which seat in grooves (not shown) in the manner hereinbefore described to again limit the amount of rotation of the movable body 112 with respect to the fixed body 118. The guide wire 49 is secured to the movable body 112 in a suitable manner such as by securing it between the fitting 51 and the extension 113. By utilizing a rotation limiting device 111 of the type shown in Figure 5, the entire second dilatation catheter 13 can be rotated with the guide wire 49 to cause rotation of the flexible tip of the catheter 13. The guide wire 49 provides torsional rigidity to ensure that rotation of the tip will occur.

It is apparent from the foregoing that there has been provided a dual dilatation catheter assembly that includes a miniature balloon dilatation catheter which can be guided by the use of a rotatable flexible tip. Because of the small size of the miniature dilatation catheter, carbon dioxide is utilized as an inflating medium for the balloon.

Safety features are provided in the device used for inflating the balloon of the miniature balloon dilatation catheter to ensure that not more than a predetermined quantity of carbon dioxide can be introduced into the body for inflation of the balloon. The size of the miniature dilatation catheter is such that it can be inserted into a conventionally sized dilatation catheter and utilized therewith.

## Claims

1. A dilatation catheter assembly for dilating a stenosis in a blood vessel, which assembly comprises a first dilatation catheter (12) having a first tubular element (16) with a first lumen (17) extending therethrough, a second tubular element extending around the first tubular element and forming an annular second lumen (19) with the first element and a first balloon (21) which is carried by the second tubular element and is in communication with the second lumen (19), the dilatation catheter assembly being characterised by a second dilatation catheter (13) extending through the first lumen (17), the second dilatation catheter having a flexible elongate tubular element (41) with a third lumen (42) extending therethrough, a second balloon (43) which is carried by the flexible tubular element (41) and is in communication with the third lumen (42) and a guiding member (49) which extends through both the third lumen, and the second balloon (43), the second balloon (43) having a collapsed diameter which is less than the inner diameter of the first lumen (17) of the first dilatation catheter (12) whereby the second dilatation catheter (13) can be inserted into and removed from the first dilatation catheter (12).

2. A catheter assembly as claimed in claim 1, characterised in that a flexible body (46) is secured to the distal end of the second balloon and disposed about and secured to the portion of the guiding member (49) wich extends out of the distal en of the second balloon (43).

3. A catheter assembly as claimed in claim 1 or 2, characterised in that the flexible elongate tubular element (41) of the second dilatation catheter (13) includes a proximal flexible metal tubular section (41a) and a distal plastic tubular section (41b) secured to the distal end of the metal tubular section, the second balloon (43) being carried by the plastic tubular section (41b).

4. A catheter assebly as claimed in any preceding claim including means (57) to rotate the guiding member (49) to facilitate the advancement of the second dilatation catheter (13).

5. A catheter assebly as claimed in claim 4, characterised in that the means (57) to rotate the guiding member (49) includes means for causing rotation of the guiding member (49) independently of the rotation of the proximal extremity of the tubular element (41) of the second dilatation catheter (13).

6. A catheter assebly as claimed in any preceding claim including means (27) for introducing a radiographic contrast liquid into the balloon (21) of the first dilatation catheter (12).

7. A catheter assembly as claimed in any preceding claim including means (86) for inflating the balloon (43) of the second dilatation catheter (13) with a predetermined quantity of fluid.

8. A catheter assembly as claimed in claim 7, characterised in that the means (86) for inflating the second balloon (43) utilises a gas and includes a cylinder (93), a piston (89) slidably mounted in the cylinder, and means for preventing the introduction of more than a predetermined quantity of gas which includes a one-way valve (96) venting to atmosphere any gas in excess of the predetermined quantity.

9. A catheter assembly as claimed in claim 8, characterised in that the one-way valve (96) is carried by the cylinder (93) and is spaced from the distal extremity of the cylinder so that a predetermined volume of the cylinder (93) corresponding to the predetermined quantity of gas extends beyond the one-way valve (96), whereby when the cylinder (93) is filled with gas and the piston (89) is advanced, the gas in the cylinder in excess of the predetermined quantity proximal of the one-way valve (96) is vented to the atmosphere through the one-way valve (96).

10. A catheter as claimed in claim 8 or 9 including a piston rod (88) extending into the cylinder (93) and being secured to the piston (89) therein and an inlet fitting (97) connected to the cylinder (93) at a proximal extremity thereof with the piston being movable in the cylinder so that it clears the inlet fitting (97) to allow the cylinder to be filled with gas.

11. A catheter assembly as claimed in claim 10 including means (101, 102) for preventig retraction of the piston (89) in the cylinder (95) beyond the inlet fitting (97) until the piston is rotated through a predetermined angle.

12. A catheter assembly as claimed in claim 11, characterised in that the means for preventing retraction of the piston (89) beyond the inlet fitting (97) until the piston is rotated through a predetermined angle includes a cooperative pin and slot arrangement carried by the piston rod (88) and the cylinder (93), the slot (101) having first, second and third portions (101a, 101b, 101c) with the first portion (101a) extending longitudinally of the piston rod, the second portion (101b) extending at a substantial angle with respect to the longitudinal axis of the piston rod and the third portion (101c) extending longitudinally of the piston rod.

13. A miniature balloon dilatation catheter (13) for dilating a stenosis in a blood vessel, with a deflated diameter not greater than 0,59 mm (0.023 inches), the catheter comprising a flexible elongate tubular element (41) with a lumen (42) extending therethrough, a balloon (43) which is carried by the flexible elongate tubular element (41) and is in communication with the lumen (42), a guiding member (49) extending through both the lumen and the balloon (43), a flexible body (46) secured to the distal end of the balloon and disposed about the portion of the guiding member (49) extending out of the distal (47) of the balloon (43) the guiding member (49) being secured to the tip (48) of said flexible body (46), and means (86) for inflating the balloon (43) with a predetermined quantity of fluid.

14. A dilatation catheter as claimed in claim 13, characterised in that the flexible elongate element (41) is formed of a proximal section of a metal tube (41a) and a distal section of a plastic tube (41b) and the balloon is integral with the plastic section.

15. A dilatation catheter as claimed in claim 13 or 14 including means (57, 111) connected to the guiding member (49) for causing limited rotation of the guiding member.

16. A dilatation catheter as claimed in claim 15, characterised in that the means (57) for causing limited rotation includes means for causing rotation of the guiding member (49) independently of the rotation of the proximal extremity of the tubular element (41).

17. A dilatation catheter as claimed in any one of claims 15, characterised in that the means (111) for causing limited rotation of the guiding member (49) includes means for causing the rotation of the guiding member and the tubular element (41).

18. A dilatation catheter as claimed in any one of claims 13 to 17, characterised in that the means (86) for inflating the balloon (43) utilises a gas and includes a cylinder (93), a piston (89) slidably mounted in the cylinder, and means for venting to the atmosphere any gas in excess of the predetermined quantity.

19. A dilatation catheter as claimed in claim 18, characterised in that the means for venting includes a one-way valve (96) which is carried by the cylinder and is spaced from the distal extremity of the cylinder so that a predetermined volume of the cylinder correponding to the predetermined quantity of the gas extends beyond the one-way valve (96), whereby when the cylinder (93) is filled with gas and the piston (89) is advanced, the gas in the cylinder in excess of the predetermined quantity is vented to the atmosphere through the one-way valve (96).

## Patentansprüche

1. Dilatationskatheteranordnung zum Erweitern einer Stenose in einem Blutgefäß, wobei die Anordnung einen ersten Dilatationskatheter (12) mit einem ersten rohrförmigen Element (16) mit einem sich dadurch erstreckenden ersten Lumen (17), ein zweites rohrförmiges Element, das sich um das erste rohrförmige Element herum erstreckt und mit dem ersten Element ein ringförmiges zweites Lumen (19) bildet, und einen ersten Ballon (21), der durch das zweite rohrförmige Element gehalten wird und mit dem zweiten Lumen (19) in Verbindung steht, aufweist, wobei die Dilatationskatheteranordnung gekennzeichnet ist durch einen zweiten Dilatationskatheter (13), der sich durch das erste Lumen (17) erstreckt, wobei der zweite Dilatationskatheter ein flexibles langgestrecktes rohrförmiges Element (41) mit einem sich dadurch erstreckenden dritten Lumen (42), einen zweiten Ballon (43), der durch das flexible rohrförmige Element (41) gehalten ist und mit dem dritten Lumen (42) in Verbindung steht, und ein Führungsglied (49), das sich sowohl durch das dritte Lumen als auch den zweiten Ballon (43) hindurch erstreckt, aufweist, wobei der zweite Ballon (43) einen kollabierten Durchmesser aufweist, der kleiner als der Innendurchmesser des ersten Lumens (17) des ersten Dilatationskatheters (12) ist, wodurch der zweite Dilatationskatheter (13) in den ersten Dilatationskatheter (12) eingesetzt und aus diesem entfernt werden kann.

2. Katheteranordnung nach Anspruch 1, dadurch gekennzeichnet, daß ein flexibler Körper (46) an dem distalen Ende des zweiten Ballons gesichert ist und um den Teil des Führungsglieds (49) herum angeordnet und daran gesichert ist, der sich aus dem distalen Ende des zweiten Ballons (43) heraus erstreckt.

3. Katheteranordnung nach Anspruch 1 oder 2, dadurch gekennzeichnet, daß das flexible langgestreckte rohrförmige Element (41) des zweiten Dilatationskatheters (13) einen proximalen flexiblen Metallrohrabschnitt (41a) und einen distalen Kunststoffrohrabschnitt (41b) umfaßt, der an dem distalen Ende des Metallrohrabschnitts gesichert ist, wobei der zweite Ballon (43) durch den Kunststoffrohrabschnitt (41b) gehalten ist.

4. Katheteranordnung nach einem der vorhergehenden Ansprüche, die Mittel (57) zum Drehen des Führungsglieds (49) umfaßt, um das Vorschieben des zweiten Dilatationskatheters (13) zu erleichtern.

5. Katheteranordnung nach Anspruch 4, dadurch gekennzeichnet daß das Mittel (57) zum Drehen des Führungsglieds (49) Mittel umfaßt, um eine Drehung des Führungsglieds (49) unabhängig von der Drehung des proximalen Endes des rohrförmigen Elements (41) des zweiten Dilatationskatheters (13) zu bewirken.

6. Katheteranordnung nach einem der vorhergehenden Ansprüche, die Mittel (27) umfaßt, um eine radiographische Kontrastflüssigkeit in den Ballon (21) des ersten Dilatationskatheters (12) einzuführen.

7. Katheteranordnung nach einem der vorhergehenden Ansprüche, die Mittel (86) umfaßt, um den Ballon (43) des zweiten Dilatationskatheters (13) mit einer vorbestimmten Fluidmenge aufzupumpen.

8. Katheteranordnung nach Anspruch 7, dadurch gekennzeichnet, daß das Mittel (86) zum Aufpumpen des zweiten Ballons (43) ein Gas verwendet und einen Zylinder (93), einen in dem Zylinder gleitbar angebrachten Kolben (89) und Mittel zum Verhindern des Einführens mehr als einer vorbestimmten Gasmenge umfaßt, welches ein Einwegventil (96) umfaßt, das jeden Gasüberschuß über der vorbestimmten Menge zur Atmosphäre entlüftet.

9. Katheteranordnung nach Anspruch 8, dadurch gekennzeichnet, daß das Einwegventil (96) durch den Zylinder (93) gehalten ist und in Abstand von dem distalen Ende des Zylinders angeordnet ist, so daß ein vorbestimmtes Volumen des Zylinders (93) sich entsprechend der vorbestimmten Gasmenge über das Einwegventil (96) hinaus erstreckt, wodurch der Gasüberschuß in dem Zylinder über der vorbestimmten Menge proximal des Einwegventils (96) durch das Einwegventil (96) zur Atmosphäre entlüftet wird, wenn der Zylinder (93) mit Gas gefüllt ist und der Kolben (89) vorgeschoben wird.

10. Katheter nach Anspruch 8 oder 9, der eine Kolbenstange (88), die sich in den Zylinder (93) erstreckt und an dem Kolben (89) darin gesichert ist, und einen Einlaßanschluß (97) umfaßt, der an den Zylinder (93) an dessen proximalem Ende angeschlossen ist, wobei der Kolben in dem Zylinder so bewegbar ist, daß er den Einlaßanschluß (97) freigibt, um eine Füllung des Zylinders mit Gas zu erlauben.

11. Katheteranordnung nach Anspruch 10, die Mittel (101, 102) umfaßt, um ein Zurückziehen des Kolbens (89) in dem Zylinder (93) über den Einlaßanschluß (97) hinaus zu verhindern, bis der Kolben um einen vorbestimmten Winkel gedreht ist.

12. Katheteranordnung nach Anspruch 11, dadurch gekennzeichnet, daß das Mittel, um ein Zurückziehen des Kolbens (89) über den Einlaßanschluß (97) hinaus zu verhindern, bis der Kolben um einen vorbestimmten Winkel gedreht ist, eine zusammenwirkende Zapfen- und Schlitzanordnung umfaßt, die durch die Kolbenstange (88) und den Zylinder (93) gehalten ist, und der Schlitz (101) erste, zweite und dritte Abschnitte (101a, 101b, 101c) aufweist, wobei der erste Abschnitt (101a) sich längs der Kolbenstange erstreckt, der zweite Abschnitt (101b) sich in einem wesentlichen Winkel im Verhältnis zur Längsachse der Kolbenstange erstreckt und der dritte Abschnitt (101c) sich längs der Kolbenstange erstreckt.

13. Miniaturballondilatationskatheter (13) zum Erweitern einer Stenose in einem Blutgefäß mit einem entleerten Durchmesser von nicht größer als 0,59 mm (0,023 Inch), wobei der Katheter ein flexibles langgestrecktes rohrförmiges Element (41) mit einem sich dadurch erstreckenden Lumen (42), einen Ballon (43), der durch das flexible langgestreckte rohrförmige Element (41) gehalten ist und mit dem Lumen (42) in Verbindung steht, ein Führungsglied (49), das sich sowohl durch das Lumen als auch den Ballon (43) erstreckt, eine flexiblen Körper (46), der an dem distalen Ende des Ballons gesichert ist und um den Teil des Führungsglieds (49) herum angeordnet ist, der sich aus dem distalen Ende (47) des Ballons (43) erstreckt, wobei das Führungsglied (49) an der Spitze (48) des flexiblen Körpers (46) gesichert ist, und Mittel (86) zum Aufpumpen des Ballons (43) mit einer vorbestimmten Fluidmenge aufweist.

14. Dilatationskatheter nach Anspruch 13, dadurch gekennzeichnet, daß das flexible langgestreckte Element (41) aus einem proximalen Abschnitt eines Metallrohrs (41a) und einem distalen Abschnitt eines Kunststoffrohrs (41b) gebildet ist und der Ballon mit dem Kunststoffabschnitt einteilig ist.

15. Dilatationskatheter nach Anspruch 13 oder 14, der Mittel (57, 111) umfaßt, die an das Führungsglied (49) angeschlossen sind, um eine begrenzte Drehung des Führungsglieds zu bewirken.

16. Dilatationskatheter nach Anspruch 15, dadurch gekennzeichnet, daß das Mittel (57) zum Bewirken begrenzter Drehung Mittel umfaßt, um eine Drehung des Führungsglieds (49) unabhängig von der Drehung des proximalen Endes des rohrförmigen Elements (41) zu bewirken.

17. Dilatationskatheter nach Anspruch 15, dadurch gekennzeichnet, daß das Mittel (111) zum Bewirken begrenzter Drehung des Führungsglieds (49) Mittel umfaßt, um eine Drehung des Führungsglieds und des rohrförmigen Elements (41) zu bewirken.

18. Dilatationskatheter nach einem der Ansprüche 13 bis 17, dadurch gekennzeichnet, daß das Mittel (86) zum Aufpumpen des Ballons (43) ein Gas verwendet und einen Zylinder (93), einen gleitbar in dem Zylinder angebrachten Kolben (89) und Mittel umfaßt, um jeden Gasüberschuß über der vorbestimmten Menge zur Atmosphäre zu entlüften.

19. Dilatationskatheter nach Anspruch 18, dadurch gekennzeichnet, daß das Mittel zum Entlüften ein Einwegventil (96) umfaßt, das durch den Zylinder gehalten wird und in Abstand von dem distalen Ende des Zylinders angeordnet ist, so daß ein vorbestimmtes Volumen des Zylinders entsprechend der vorbestimmten Gasmenge sich über das Einwegventil (96) hinaus erstreckt, wodurch der Gasüberschuß über der vorbestimmten Menge in dem Zylinder durch das Einwegventil (96) zur Atmosphäre entlüftet wird, wenn der Zylinder (93) mit Gas gefüllt ist und der Kolben (89) vorgeschoben wird.

## Revendications

1. Ensemble de cathéters à dilatation destiné à dilater une sténose sans un vaisseau sanguin, lequel ensemble comprend un premier cathéter à dilatation (12) possédant un premier élément tubulaire (16) qui présente une première chambre (17), un deuxième élément tubulaire qui entoure le premier élément tubulaire et forme une deuxième chambre annulaire (19) en combinaison avec le premier élément, et un premier ballonnet (21) qui est porté par le deuxième élément tubulaire et est en communication avec la deuxième chambre (19), l'ensemble cathéter à dilatation étant caractérisé par un deuxième cathéter à dilatation (13) enfilé dans la première chambre (17), le deuxième cathéter à dilatation possédant un élément tubulaire flexible de grande longueur (41) qui présente une troisième chambre (42), un deuxième ballonnet (43) qui est porté par l'élément tubulaire flexible (41) et est en communication avec la troisième chambre (42), et un élément de guidage (49) enfilé dans la troisième chambre et dans le deuxième ballonnet (43), le deuxième ballonnet (43) possédant un diamètre à l'état dégonflé qui est inférieur au diamètre intérieur de la première chambre (17) du premier cathéter à dilatation (12), de sorte que le deuxième cathéter à dilatation (13) peut être inséré dans le premier cathéter à dilatation (12) et en être extrait.

2. Ensemble de cathéters selon la revendication 1, caractérisé par le fait qu'un corps flexible (46) est fixé à l'extrémité distale du deuxième ballonnet (43) et disposé autour et fixé à la portion de l'élément de guidage (49) qui émerge de l'extrémité distale du deuxième ballonnet (43).

3. Ensemble de cathéters selon l'une des revendications 1 ou 2, caractérisé par le fait que l'élément tubulaire flexible de grande longueur (41) du deuxième cathéter à dilatation (13) comprend une section proximale tubulaire métallique flexible (41) et une section distale tubulaire plastique (41b) fixée à l'extrémité distale de la section tubulaire métallique, le deuxième ballonnet (43) étant porté par la section tubulaire plastique (41b).

4. Ensemble de cathéters selon l'une des revendications précédentes, caractérisé en ce qu'il comprend des moyens (57) servant à faire tourner l'élément de guidage (49) pour faciliter l'avancement du deuxième cathéter à dilatation (13).

5. Ensemble de cathéters selon la revendication 4, caractérisé par le fait que les moyens (57) servant à faire tourner l'élément de guidage (49) comprennent des moyens servant à provoquer la rotation de l'élément de guidage (49) indépendamment de la rotation de l'extrémité proximale de l'élément tubulaire (41) du deuxième cathéter à dilatation (13).

6. Ensemble de cathéters selon l'une des revendications précédentes, caractérisé en ce qu'il comprend des moyens (27) permettant d'introduire un liquide de contraste radiographique dans le ballonnet (21) du premier cathéter à dilatation (12).

7. Ensemble de cathéters selon l'une des revendications précédentes, caractérisé en ce qu'il comprend des moyens (86) servant à gonfler le ballonnet (43) du deuxième cathéter à dilatation (13) avec une quantité de fluide prédéterminée.

8. Ensemble de cathéters selon la revendication 7, caractérisé par le fait que les moyens (86) servant à gonfler le deuxième ballonnet (43) utilisent un gaz et comprennent un cylindre (93), un piston (89) monté coulissant dans le cylindre, et des moyens servant à empêcher l'introduction d'une quantité de gaz supérieure à une quantité prédéterminée, qui comprennent un clapet unidirectionnel (96), qui met à l'échappement sur l'atmosphère le gaz éventuellement présent en excès de la quantité prédéterminée.

9. Ensemble de cathéters selon la revendication 8, caractérisé par le fait que le clapet unidirectionnel (96) est porté par le cylindre (93) et est espacé de l'extrémité distale du cylindre de telle manière qu'il subsiste au-delà du clapet unidirectionnel (96) un volume prédéterminé du cylindre (93) qui correspond à ladite quantité de gaz prédéterminée, de sorte que, lorsque le cylindre (93) est rempli de gaz et qu'on fait avancer le piston (89), le gaz contenu dans le cylindre en excès de la quantité prédéterminée et qui est proche du clapet unidirectionnel (96) est mis à l'échappement dans l'atmosphère à travers le clapet unidirectionnel (96).

10. Ensemble de cathéters selon l'une des revendications 8 ou 9, caractérisé en ce qu'il comprend une tige de piston (88) qui pénètre dans le cylindre (93) et est fixée au piston (89) à l'intérieur de ce cylindre, et un raccord d'entrée (97) relié au cylindre (93) à une extrémité proximale de celui-ci, le piston pouvant se déplacer dans le cylindre de manière à démasquer le raccord d'entrée (97) pour permettre au cylindre de se remplir de gaz.

11. Ensemble de cathéters selon la revendication 10, caractérisé en ce qu'il comprend des moyens (101, 102) qui empêchent de tirer le piston (89) dans le cylindre (93) au-delà du raccord d'entrée (97) avant d'avoir tourné le piston d'un angle prédéterminé.

12. Ensemble de cathéters selon la revendication 11, caractérisé par le fait que les moyens servant à empêcher de tirer le piston (89) au-delà du raccord d'entrée (97) avant d'avoir tourné le piston d'un angle prédéterminé, comprennent un dispositif formé d'un ergot et d'une fente qui coopèrent entre eux, qui sont portés, l'un par la tige (88) et l'autre par le cylindre (93), la fente (101) possédant des première, deuxième et troisième portions (101a, 101b, 101c), la première portion (101a) s'étendant dans la direction longitudinale de la tige de piston, la deuxième portion (101b) formant un angle notable avec l'axe longitudinal de la tige du piston et la troisième portion (101c) s'étendant dans la direction longitudinale de la tige du piston.

13. Cathéter à dilatation (13) à ballonnet miniature, destiné à dilater une sténose dans un vaisseau sanguin, possédant un diamètre à l'état dégonflé non supérieur à 0,59 mm, le cathéter comprenant un élément tubulaire flexible de grande longueur (41) qui présente une chambre (42), un ballonnet (43) qui est porté par l'élément tubulaire flexible de grande longueur (41) et qui est en communication avec la chambre (42), un élément de guidage (49) enfilé dans la chambre et dans le ballonnet (43), un corps flexible (46) fixé à l'extrémité distale (47) du ballonnet et disposé autour de la portion de l'élément de guidage (49) qui émerge de l'extrémité distale du ballonnet (43), l'élément de guidage (49) étant fixé à la pointe (48) dudit corps flexible (46), et des moyens (86) servant à gonfler le ballonnet (43) à l'aide d'une quantité de fluide prédéterminée.

14. Cathéter à dilatation selon la revendication 13, caractérisé par le fait que l'élément flexible de grande longueur (41) est formé d'une section proximale faite d'un tube métallique (41a) et d'une section distale faite d'un tube plastique (41b) et que le ballonnet est d'une seule pièce avec la section plastique.

15. Cathéter à dilatation selon l'une des revendications 13 ou 14, caractérisé en ce qu'il comprend des moyens (57, 111) reliés à l'élément de guidage (49) pour commander une rotation limitée de ce dernier.

16. Cathéter à dilatation selon la revendications 15, caractérisé par le fait que les moyens (57) servant à provoquer une rotation limitée comprennent des moyens servant à provoquer une rotation de l'élément de guidage (49) indépendamment de la rotation de l'extrémité proximale de l'élément tubulaire (41).

17. Cathéter à dilatation selon la revendication 15, caractérisé par le fait que les moyens (111) servant à provoquer une dilatation limitée de l'élément de guidage (49) comprennent des moyens servant à provoquer la rotation de l'élément de guidage et de l'élément tubulaire (41).

18. Cathéter à dilatation selon l'une quelconque des revendications 13 à 17, caractérisé par le fait que les moyens (86) servant à gonfler le ballonnet (43) utilisent un gaz et comprennent un cylindre (93), un piston (89) monté coulissant dans le cylindre et des moyens servant à laisser échapper dans l'atmosphère le gaz éventuellement en excès de la quantité prédéterminée.

19. Cathéter à dilatation selon la revendication 18, caractérisé par le fait que les moyens servant à mettre à l'échappement sur l'atmosphère comprennent un clapet unidirectionnel (96) qui est porté par le cylindre et qui est espacé de l'extrémité distale du cylindre de telle manière qu'un volume prédéterminé du cylindre qui correspond à la quantité de gaz prédéterminé s'étende au-delà du clapet unidirectionnel (96), de sorte que, lorsque le cylindre (93) est rempli de gaz et que l'on fait avancer le piston (89), le gaz contenu dans le cylindre en excès de la quantité prédéterminée est mis à l'échappement sur l'atmosphère à travers le clapet unidirectionnel (96).

FIG.-6

FIG.-1A

FIG.-5

FIG.-1B

FIG.-4

FIG.-3

FIG.-2

EP 0 213 748 B1